# EUROPEAN PATENT APPLICATION

(11) **EP 1 033 136 A1**
(43) Date of publication of application: **06.09.2000**
(21) Application number: 99926233.0
(22) Date of filing: 21.06.1999
(51) Int. Cl.: A61K 39/395, A61L 2/04

(54) **THE PROCESS OF PREPARING IMMUNOGLOBULIN FOR INTRAVENOUS INJECTION BY VIRUSES DOUBLE-STERILIZED WITHOUT ADDING ANY PROTECTANT**

(30) Priority: 25.06.1998 CN 98112108
(71) Applicant: Chengdu Shuyang Pharmaceutical Factory, Chengdu, Si Chuan Province 610214 (CN)
(72) Inventor: CHEN, Aimin, Chengdu, Si Chuan Province 610214 (CN); FAN, Shaowen, Chengdu, Si Chuan Province 610214 (CN); ZHOU, Chao, Chengdu, Si Chuan Province 610214 (CN)
(74) Representative: Schrell, Andreas, Dr.
(86) International application number: PCT/CN99/00081
(87) International publication number: WO 99/66955

(57) **Abstract**

The present invention related to the process for preparing biological agent. The viruses are double-sterilized in the process using the combination of Pasteurization and incubation at low pH without using any protectant. The viruses are efficiently killed not only, the completeness and native biological activity of immunoglobulin and purity and safety of the product are also kept. The form of final product is lipuid instead of lyophilized powder. 5-10 % glucose is added to the final product in order to keep the osmotic equilibrium of solution and avoid the undesired effects after administration.

## Description

### FIELD OF THE INVENTION

The present invention relates to a process for preparing biological product, especially for preparing immunoglobulin for intravenous injection (IVIG) by viruses double-sterilized without using any protectant.

### BACKGROUND OF THE INVENTION

The production of immunoglobulin started in 1949. More than 30 kinds of IVIG produced by 29 manufacturers in 12 countries, have appeared in international market since the invention of IVIG in the 1960s.

The human placental blood IVIG first came out in China in 1985, and its regulations of manufacture and assay have been recorded in "The China National Regulations of Biological Products". In 1992, the pilot-plant production of lyophilized, low pH IVIG was approved by Ministry of the Health of P.R. China.

There have been many reports concerning the cases of epidemic type C hepatitis due to repeated injection with large dose of IVIG. More and more evidence has proved that its safety is difficult to ensure without procedure of viruses sterilization in the course of IVIG production. Since 1990s, "viruses sterilization", as an essential procedure in the production of IVIG has been regulated by many countries in the world.

Pasteurization is the earliest method for studying viruses sterilization, i.e. heat sterilization for 60°C, 10 hours.

Owing to the rapid spread of AIDS, the method which destroys virus lipid-containing envelope using detergent, has been developed for HIV. The methods such as low pH incubation, Dry heat, Irradiation and Filtration are also developed one after another.

Although above-mentioned methods of viruses sterilization are able to sterilize for HIV, HBV, HCV and some common viruses pathogenic factors, there are some differences between the various methods for the ability of different viruses sterilization. Particularly, there is some protection for virus in the presence of protectant, and a trace amount of viruses may survive in the product. The survived organic solvents are also a problem in the detergent sterilization, which influence not only the purity of the product, but also the undesired effects produced when administered intravenously.

At present, most of IVIG products are of lyophilized preparation in which saccharose serves as protectant and excipient.

According to the recent literature's reports (Ref. Murphy P. et al., J-Med- Virol, 41(1): 61-64,1993), although the presence of protectant has some effect for keeping natural activity of protein, it can yield a certain protection for virus. At the same time, it was reported that use of saccharose as protectant had led to failure of acute kidney function in several cases. (Ref. Winward DB et al., Pharmacotherapy, 15(6): 765-722,1995; Hifenhaus L. & Nowak T., Vox Sang 67 (Suppl.) 1:62-66,1994; Horowitz B.et al., Blood, 86(11): 4331-4336, 1995).

To summarize above descriptions, clinical side effects of lyophilized IVIG is greater than liquid products obviously, because the polymerization took place in part of IVIG molecule during the lyophilization, besides, lyophilized preparation is not favorable for large-scale production.

### SUMMARY OF THE INVENTION

The object of this invention is to overcome the deficiency in the existing techniques, and provided a process of preparing IVIG by viruses double-sterilized without any protectant, i.e. using viruses-sterilizing method with two different mechanisms, it will reach to complete sterilization for virus, and thus, IVIG can be used in the clinics more effectively and safely.

### DETAILED DESCRIPTION OF THE INVENTION

### The first step: Pasteurilization

Taking Cohn's component II (F II) as raw material and dissolving it in 2-10-fold ice distilled water, adjust it to pH 3.5-5.0 with 0.2-2.0 mmol/L acetic acid solution, and then ulrafilter, dealcohol and desalt to sodium ion concentration of 1-10 mmol/L with a molecular weight 10-100 kDa ultrafilter membrane. Adjust IVIG concentration to 0.5-2% before bottling, fill in gaseous CO₂ till the internal pressure reaches 0.7-200 kPa; seal bottles in the absence of any protectant and sterilize at 60±1°C for 10 hours.

### The second step: Incubation treatment at low pH.

F II solution after pasteurilization was cleaved and purified with 10-100 kDa ultrafilter membrane. If purity of IVIG is still <97%, its purity can be increased through gel adsorption and purification, then concentrate IVIG to 5-10%, adjust pH 4.1±0.3, remove bacteria by filter, and place it at room temperature for 21 days. Adjust osmotic pressure by adding 5-10% glucose, determination of semi-finished product and biological test are carried out after removing bacteria. If the semi-finished product determination is qualified, and remove bacteria by filter, bottling 25ml/bottle or 50ml/bottle.

Overall quality control test will be carried out according to the regulation of IVIG.

In order to ensure effectively the quality and safety for IVIG, the test of IVIG produced according to the above-mentioned methods will be carried out. (The test will be undertaken by West China University of Medical Sciences). Using indicator virus as VSV Polio-I and Sindbis, Viruses sterilization using Pasteurilization, low pH sterilization and virus double-sterilized method (i.e. this invention) and the results are shown on Table 1, Table 2 and Table 3.

### 1. Pasteurilization

**Table 1**

| Sterilizing effect on three kinds of virus at 60°C | | | | | | |
|---|---|---|---|---|---|---|
| Sterilizing time (h) | VSV Log TCID₅₀/0.1 ml | | Polio-I Log TCID₅₀/0.1 ml | | Sindbis Log PFU/ml | |
| | control sample | treat sample | control sample | treat sample | control sample | treat sample |
| 0 | 6 | | 6.5 | | 4×10⁶ | |
| 1 | 6 | 4 | 6.5 | 2 | 4×10⁶ | 10⁴ |
| 4 | 6 | 3 | 6.3 | - | 4×10⁶ | <10⁴ |
| 5 | 5.8 | 2 | 6.3 | - | 4×10⁶ | <10³ |
| 7 | 5.8 | 1 | 6.3 | - | 4×10⁶ | <10² |
| 10 | 5.8 | - | 6 | - | 4×10⁶ | - |

As can be seen from Table 1, Sterilizing effect of Pasteulization on Polio-I virus is considerably remarkable, but less for VSV and slightly poor for Sindbis.

### 2. Low pH Sterilization

**Table 2**

| Sterilizing effect on two kinds of virus at pH 4.0, 23°C | | | | |
|---|---|---|---|---|
| Time (day) | VSV (Log TCID₅₀/0.1 ml) | | Sindbis (Log PFU/ml) | |
| | control sample | treat sample | control sample | treat sample |
| 0 | 6.5 | | 4×10⁶ | |
| 3 | 6.5 | 1 | 4×10⁵ | <10⁴ |
| 7 | 8 | - | 3.5×10⁶ | <10³ |
| 14 | 6 | - | 3.5×10⁶ | <10⁰ |
| 21 | 5.5 | - | 3.5×10⁴ | - |

The results given in Table 2, indicated that VSV is very sensitive to acidic environment of pH 4.0, and easy to be inactivated. The 7^{th} day after treatment, virus has no activity, while Sindbis virus is less sensitive to pH 4.0. Until 21st day after treatment, virus was completely inactivated.

### 3. The viruses double-sterilization (this invention)

**Table 3**

| Sterilizing effect of viruses Double-sterilization on three kinds of virus | | | | | | | |
|---|---|---|---|---|---|---|---|
| | sterilizing time (h) | VSV Log TCID₅₀/0.1ml | | Polio-I Log TCID₅₀/ml | | Sindbis Log PFU/ml | |
| | | Control Sample | treat sample | control sample | treat sample | control sample | treat sample |
| 60°C | 0 | 6 | | 6.5 | | 4×10⁶ | |
| | 5 | 6 | 2 | 6.5 | - | 4×10⁶ | 10³ |
| heating | 10 | 6 | - | 6.3 | - | 4×10⁶ | - |
| stored at | 7 days | 5.5 | - | 6 | - | 3.5×10⁶ | - |
| pH 4.0 | 14 days | 5.5 | - | 6 | - | 3.5×10⁶ | - |
| 23°C | 21 days | 5 | - | 5.5 | - | 3.5×10⁶ | - |

Table 3 shows that three kinds of indicator virus has been inactivated by the pasteurization for 10 hrs. The product will reach to the best safety if low pH sterilization was performed continuously.

We sent the product prepared by this invention to the National Institute for Control of Pharmaceutical and Biological Products and AIDS Detection and Confirmation Laboratory of the People's Liberation Army (PLA) for detection. The result is shown in Table 4 and Table 5.

### 1.Pasteurization

**Table 4**

| Sterilizing effect on four kinds of virus at 60°C | | | | | | | | |
|---|---|---|---|---|---|---|---|---|
| Time (h) | VSV Log TCID₅₀/0.1ml | | Polio- I Log TCID₅₀/0.1ml | | HIV Log TCID₅₀/0.1ml | | Sindbis Log PFU/ml | |
| | control sample | treat sample | control sample | treat sample | control sample | treat sample | control sample | treat sample |
| 0 | 7.00 | 5.00 | 6.00 | 3.13 | 6.00 | 5.77 | - | 6.63 |
| 0.5 | ≤-0.50 | ≤0.50 | ≤-0.50 | ≤0.50 | - | - | - | ND |
| 1 | ≤-0.50 | ≤0.50 | ≤-0.50 | ≤0.50 | - | - | - | ND |
| 4 | ≤-0.50 | ≤0.50 | ≤-0.50 | ≤0.50 | - | - | - | ND |
| 5 | ≤-0.50 | ≤0.50 | ≤-0.50 | ≤0.50 | - | - | - | - |
| 6 | ≤-0.50 | ≤0.50 | ≤-0.50 | ≤0.50 | - | - | - | ND |
| 10 | ≤-0.50 | ≤0.50 | ≤-0.50 | ≤0.50 | ≤5.80 | <2.00 | - | - |

In this experiment, virus minimal defection limit: ≤0.50 Log TCID₅₀/0.1 ml. Titer of Sindbis basal virus: 7.70 Log PFU/ml. ND = No virus detected.

The conclusion of detection is:
Detected samples were treated by Pasteunization (heating at 60°C for 10 hrs). It has marked sterilization for indicator virus VSV, HIV, Sindbis and Polio-I. After heating for 30 minutes, virus titers were decreased below detection limit Viruses decreased were VSV: ≥4.50-4.63, Polio-I: ≥ 2.63-3.63, HIV: ≥3.77-4.17 (Unit Log TCID₅₀/0.1ml), and Sindbis: ≥ 6.32-6.41 Log PFU/ml, respectively. For the sample which heating 6 hrs, 10 hrs, three generations of blind passage were negative.

### 2.Low pH Sterilization

**Table 5**

| Sterilizing effect on three kinds of virus incubation at low pH and room temperature | | | | | | |
|---|---|---|---|---|---|---|
| Time (day) | VSV Log TCID₅₀/0.1ml | | HIV* Log TCI₅₀/0.1ml | | Sindbis Log PFU/ml | |
| | Acid | Neutral | Acid | Neutral | Acid | Neutral |
| 0 | 3.88 | 6.38 | 5.77 | 6.00 | 3.75 | 7.34 |
| 3 | 3.13 | 6.13 | - | - | ND | 7.09 |
| 7 | 1.75 | 5.88 | <2.00 | 5.80 | ND | 7.09 |
| 14 | ≤0.50 | 5.18 | - | - | ND | 6.60 |
| 21 | ≤0.50 | 4.88 | - | - | ND | 6.23 |

In this experiment, virus minimal detection limit: ≥0.50 Log TCID₅₀/0.1ml
* The sample after Pasteurization
Titer of Sindbis basal virus: 8.35 Log PFU/ml.
DN = No virus detected

The conclusion of detection is:

The detected samples were incubated at pH 4.0 ± 0.2, 22-24°C for 21 days. Sterilizing VSV ≥ 5.88-6.63 Log TCID₅₀/0.1ml, HIV ≥ 3.77-4.17, and Sindbis ≥ 7.33-7.74 Log PFU/ml. Three generations of blind passage were negative.

To sum up the results of the above-mentioned experiments, the following conclusions were drawn out:
1. The Pasteurization at 60°C 10 hr is capable of sterilizing four kinds of indicator virus, especially more effective for VSV and Polio-I virus.
2. The low pH incubation is capable of sterilizing effectively for VSV, HIV and Sindbis virus. It appears very strong sterilizing effect on VSV, HIV & Sindbis, and completely inactivated at 7 days.
3. For viruses double-sterilization, after Pasteurization for 10 hrs, again store it at pH 4.0, room temperature for 21 days, all of indicator virus will be inactivated more steadily. The safety of blood product will be guaranteed.

To compare with the existing techniques, the advantages of IVIG by the manufacturing process in this invention are as follows:
1.No any protectant is added in the process. It can effectively inactivated viruses, keeping integrity of IVIG molecule and natural biological activity, purity and safety of the product, and having good stability. The virus-sterilizing effect of the product and the overall quality control have been verified and examined by the Ministry of Health of P.R. China and accorded with the standards of the National Regulation of Biological Products, 1995 edition.
2. The liquidus preparation is selected for preparing IVIG, rather than lyophilized powder in the process. It shortens the period of production, lowers energy consumption, and is favourable for large-scale production. The quality of the product is stable and reliable.
3. 5-10% glucose is added to the product for adjusting osmotic pressure equilibrium of solution, and preventing some patients from the occuring of undesired effects after administration.

### EXAMPLES

### Example 1

The process for preparing human blood IVIG by viruses double-sterilized without Protectant.

### The first step Pasteurization:

16.5kg F II precipitate was dissolved in 165 liter distilled water (0°C), adjusted the pH value to 3.5 with 1mmol/L acetic acid solution, and ultrafiltered to dealcohol, desalt to sodium ion concentration of 1.2mmol/L with 100 kDa ultrafiltration membrane. Adjusted IG concentration to 1.5%; bottling; sealed bottle under internal pressure 100kPa by charging CO₂,; sterilized at 60 ± 1°C for 10 hrs.

### The second step incubation treatment at low pH:

F II solution after the Pasteurization was cleaved and purified with a 100 kDa ultrafilter membrane. If purity of IG is still < 97%, its purity can be increased to $ge$$ 98% using DEAE-Sephadex A-50 gel adsorption. Then, concentrated the IG to 5%, adjusted the pH value to 4.1, filtered and removed bacteria. Stored it for 21 days, then added 5% glucose. After remove bacteria, sent semi-finished products for physical and chemical determination, pyrogen test and sterility test. If the semi-finished product determination is up to standard, filter and remove bacteria; bottling 25ml/bottle or 50ml/bottle. The overall quality control test were carried out according to the regulation of human blood IG.

### Example 2

The process for preparing human blood IVIG by viruses double-sterilized without proctectant.

### The first step Pasteurization:

15kg F II precipitate was dissolved in 150 liter distilled water (0°C), adjusted the pH value to 5.0 with 0.2mmol/L acetic acid solution and ultrafiltered to dealcohol, desalt to sodium ion concentration of 10mmol/L by 10 kDa ultrafilter membrane, adjusted IG contentration to 2%, bottling; sealed bottle under internal pressure 0.7 kPa by charging CO₂; sterilized at 60 ± 1°C for 10 hrs.

### The second step incubation treatment at low pH:

F II solution after the Pasteurization was cleaved arid purified with a 10 kDa ultrafilter membrane. If purity of IG is still < 97%, its purity can be increased to ≥ 98%, using DEAE-Sephadex A-50 gel adsorption. Then concentrated IG to 8%, adjusted the pH value to 4.1, filtered and removed bacteria. Stored it for 21 days, added 10% glucose. The latter procedures are same as example 1.

### Example 3

The process for preparing human blood IVIG by viruses double-sterilized without protectant:

### The first step Pasteurization:

16.5kg F II precipitate was dissolved in 165 liter distilled water (0°C), adjusted the pH value to 4.2 with 2.0mmol/L acetic acid solution and ultrafiltered to dealcohol, desalt to sodium ion concentration of 5mmol/L by 50 kDa ultrafilter membrane, adjusted IG concentration to 0.5%; bottling; sealed bottle under internal pressure 200kPa by charging CO₂; sterilized at 60 ± 1 °C for 10 hrs.

### The second step incubation treatment at low pH:

F II solution after the Pasteurization was cleaved and purified with a 50 kDa ultrafilter membrane. If purity of IG is still < 97%, its purity can be increased to ≥ 98% using DEAB-Sephadex A-50 gel adsorption. Then concentrated IG to 10%; adjusted the pH value to 4.1, filtered and removed bacteria. Stored it for 21 days, added 8% glucose. The latter procedures are same as Example 1.

## Claims

1. A process of preparing immunoglobulin for intravenous injection by viruses double-sterilized without adding any protectant, which is characterized by the first step-Pasteurization: taking Cohn's component II as a raw material and dissolving it in 2-10 folds ice distilled water, adjusting it to pH3.5-5.0 with 0.2-2.0mmol/L acetic acid and then ultrafiltraing dealcohol and desalt to sodium ion concentration of 1-10mmol/L by a molecualr weight 10-100 kDa ultrafilter membrane, after that, adjusting IG concentration to 0.5-2%; bottling; filling gaseous CO₂ fill the pressure reaches 0.7-200 kPa, sealing bottle in the absence of any protectant and sterilizing at 60 ± 1°C for 10 hrs; and the second step - incubation treatment at low pH:
clearing and purifying FII solution after Pasteurization with 10-100 kDa ultrafilter membrane, then concentrateing it to 5-10% IG, adjusting pH to 4.1 ± 0.3, fitering and removing bacteria; finally storing it at room temperature for 21 days, then adding 5-10% glucose for adjusting the osmotic pressure equilibrium of solution.

2. A process of claim 1, which is characterized by the second step-incubation treatment at low pH, wherein said F II solution after Pasteurization is cleaved and purified with a 10-100 kDa ultrafilter membrane, when purity of IG is < 97%; it's purity can be improved by gel adsorption.
